(19) Europäisches Patentamt
European Patent Office
Office européen des brevets

(11) Veröffentlichungsnummer: **0 169 365**
**A1**

(12) **EUROPÄISCHE PATENTANMELDUNG**

(21) Anmeldenummer: 85107424.5

(22) Anmeldetag: 15.06.85

(51) Int. Cl.[4]: **C 08 L 83/07**
//A61K6/10, (C08L83/07, 83:05)

(30) Priorität: 28.06.84 DE 3423823

(43) Veröffentlichungstag der Anmeldung:
29.01.86 Patentblatt 86/5

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Schwabe, Peter, Dr.
Dudweiler Strasse 17
D-5090 Leverkusen(DE)

(72) Erfinder: Wrobel, Dieter, Dr.
Jakob-Böhme-Strasse 11
D-5000 Koeln 80(DE)

(54) **Verwendung von Compounds aus hochdispersen aktiven Füllstoffen und Silikonpolymeren für Silikonpasten und Silikonabformmassen sowie solche Massen.**

(57) Additionsvernetzende Massen auf Polysiloxanbasis, enthaltend

a) ein Organopolysiloxan mit zwei oder mehreren Vinylgruppen im Molekül,

b) ein Organopolysiloxan ohne reaktive Gruppen,

c) ein Organohydrogenpolysiloxan als Vernetzer,

d) einen Katalysator zur Beschleunigung der Additionsreaktion

e) gegebenenfalls anorganische Füllstoffe, gegebenenfalls

f) organische Strukturbildner und gegebenenfalls

g) Farbstoffe,

dadurch gekennzeichnet, daß sie zusätlich Compounds von hochdispersen Füllstoffen in Silikonölen enthalten, wobei Komponente a) ganz oder teilweise im Compound enthalten win kann.

EP 0 169 365 A1

Croydon Printing Company Ltd

BAYER AKTIENGESELLSCHAFT 5090 Leverkusen, Bayerwerk
Konzernverwaltung RP
Patentabteilung Sft/ABc

# Verwendung von Compounds aus hochdispersen aktiven Füllstoffen und Silikonpolymeren für Silikonpasten und Silikonabformmassen, sowie solche Massen

Die vorliegende Erfindung betrifft pastenförmige Silikonmassen, welche Compounds von hochdispersen aktiven Füllstoffen in Silikonölen enthalten. Die Silikon-Pasten eignen sich insbesondere zur Herstellung genauer Abformungen bezahnter, teilbezahnter und unbezahnter Kiefer sowie zur Vervielfältigung von Gipsmodellen. Bei den Silikon-Pasten handelt es sich vorzugsweise um an sich bekannte kalt vulkanisierende Zweikomponenten-Silikonkautschuksysteme, bei denen zwei getrennt aufbewahrte Pasten vermengt werden, die dann nach ca. 2 - 5 Minuten (Abformmassen) bzw. 5 - 25 Minuten (Dubliermassen) bei Raumtemperatur vernetzen.

Silikon-Pasten zur Zahnabformung sind weit verbreitet. Im allgemeinen bestehen sie aus einem mit Füllstoffen vermischten Silikonöl auf Basis eines endständige Hydroxylgruppen aufweisenden Polydimethylsiloxans, welches je nach Applikationsmethode in verschiedenen Konsistenzen erhältlich ist, und einer flüssigen oder

pastenförmigen Härterkomponente, die ein Metallsalz einer Monocarbonsäure als Katalysator und einen Kieselsäureester als Vernetzer enthält.

Diese beiden Komponenten werden vor der Anwendung miteinander gemischt und vernetzen bei Raumtemperatur innerhalb von 2-5 Minuten infolge einer Polykondensationsreaktion. Hierbei entstehen neben dem vernetzten Silikongummi noch geringe Mengen Alkohol, die langsam aus dem Gummi herausdiffundieren und eine lineare Schrumpfung hervorrufen, was bei den Abformungen zu Ungenauigkeiten führt.

Wesentlich geringer ist die Schrumpfung bei den erst seit wenigen Jahren bekannten Vinylsilikon-Abformmassen. Diese Massen bestehen aus zwei Pasten: einer Silikonöl, Füllstoff und Vernetzer enthaltenden Basispaste und einer Katalysatorpaste aus Silikonöl, Füllstoff und Katalysator. Bei dem Silikonöl handelt es sich um ein endständige Vinylgruppen aufweisendes Polydimethylsiloxan, der Vernetzer enthält die reaktiven SiH-Gruppen und der Katalysator besteht aus einem Platinkomplex. Zu der größeren Dimensionsgenauigkeit der Abformung kommen bei diesem System als weitere Vorteile noch die bessere Dosierbarkeit von Basis- und Katalysatorpaste infolge gleicher Pastenviskosität und des abgestimmten Mischungsverhältnisses von ca. 1:1 hinzu sowie die völlige Geschmack- und Geruchlosigkeit der beiden Pasten.

Entsprechend den Abformmassen in der Zahnarztpraxis werden im zahntechnischen Labor die Dubliermassen zur Vervielfältigung der Gipsmodelle eingesetzt. Weit verbreitet sind zu diesem Zweck Massen auf Agar-Agar-Basis, deren Nachteile jedoch u.a. die Notwendigkeit einer genauen Temperaturführung und eine lange Wartezeit bis zur Weiterverarbeitung sind, und die nur eine begrenzte Lagerfähigkeit infolge Wasserverdunstung aufweisen. Diese Mängel besitzen Massen auf Basis der additionsvernetzenden Vinylsilikone nicht.

Da in der Zahnheilkunde verschiedene Techniken zur Abformung bezahnter, teilbezahnter und unbezahnter Kiefer und der Schleimhaut angewendet werden, ist ein Angebot von Abformmassen in verschiedenen Viskositätsklassen notwendig, z.B. niedrig-, mittel-, hochviskose und knetbare Massen. Die Massen bestehen jeweils aus einer Basispaste und einer speziell auf die erforderliche Verarbeitungszeit eingestellten Katalysatorpaste.

Dubliermassen sollen niedrigviskos sein, damit sie das zu vervielfältigende Gipsmodell vollständig und blasenfrei umfließen. Im allgemeinen werden Dubliermassen in Portionspackungen konfektioniert, so daß ein mengenmäßiges Mischungsverhältnis von Basis- zu Katalysatorpaste von 1:1 nicht unbedingt erforderlich ist.

Problematisch ist bei den Abform- und Dubliermassen auf Basis additionsvernetzender Vinylsilikone die Auswahl der Füllstoffe. Spuren von Schwefel und Schwermetallen

wie Eisen, Nickel und Blei wirken als Katalysatorgift gegenüber dem Platinkatalysator; bei längerer Lagerzeit wird das Platin durch diese Schwermetalle aus seiner Komplexverbindung gedrängt, die Pasten ändern ihre Reaktivität und damit auch die Eigenschaften des Silikongummis. Feuchtigkeit und/oder SiOH-Gruppen an der Füllstoffoberfläche, speziell wiederum bei sauer oder basisch reagierenden Füllstoffen, reagieren mit den SiH-Gruppen des Vernetzers unter Bildung von Wasserstoff. Dies bedeutet, daß sich bei längerer Lagerzeit die Basispasten enthaltende Tuben aufblähen, der Silikongummi enthält feine Blasen und hat daher schlechtere mechanische Eigenschaften. Das später hergestellte Gipsmodell ist durch feine Wasserstoff-Bläschen oberflächlich geschädigt und damit unbrauchbar.

Am gebräuchlichsten sind als Füllstoffe in der Praxis Quarz- und Cristobalitmehle und Calciumcarbonat mit beladener oder auch unbeladener Oberfläche.

In den hoch-, mittel- und niedrigviskosen Abformmassen haben diese Füllstoffe den Nachteil, daß sie infolge ihrer hohen Dichte zur Sedimentation neigen. Im allgemeinen wird dies durch Zusatz von gefällter oder pyrogen hergestellter Kieselsäure mit beladener oder unbeladener Oberfläche weitgehend verhindert. Die mit diesen Füllstoffen hergestellten Massen neigen jedoch zur Viskositätsänderung bei der Lagerung und ergeben einen wenig reißfesten Gummi. Für die Dubliermassen ist dieses Füllstoff-Konzept wenig geeignet, da der aus solchen feinteilige Füllstoffe enthaltenden Massen hergestellte Gummi nicht weich, dehnbar und reißfest genug ist.

Durch den erfindungsgemäßen Einsatz von Compounds von hochdispersen aktiven Füllstoffen in Silikonölen, welche in der DE-OS 25 35 334 beschrieben sind, erhält man hinsichtlich Viskosität und Sedimentation lagerstabile Pasten, die zu Gummis mit guter Reißfestigkeit und Flexibilität bei hohem Rückstellvermögen vernetzen. Bei den Abformmassen setzt man vorzugsweise neben den Compounds noch weitere konventionelle Füllstoffe, wie Calciumcarbonat, Cristobalit- und Quarzmehl mit beladener oder unbeladener Oberfläche zu, um eine höhere Gummihärte zu erzielen und die Massen billiger zu machen, sowie bevorzugt hydriertes Rizinusöl,um eine gewisse Strukturviskosität zu bewirken. Um eine gute Fließfähigkeit der Massen und Weichheit, Flexibilität und hohes Rückstellvermögen des Gummis zu erreichen, setzt man den Dubliermassen neben den erfindungsgemäß zu verwendenden Compounds vorzugsweise noch niedrigviskose trimethylsiloxyendgestoppte Silikonöle zu. Strukturbildner und zusätzliche konventionelle Füllstoffe werden bei Dubliermassen in der Regel nicht zugesetzt.

Gegenstand der Erfindung sind somit bei Umgebungstemperatur härtbare Massen auf Polysiloxanbasis, die nach dem Additionsverfahren vernetzen, enthaltend

a) ein Organopolysiloxan mit zwei oder mehr Vinylgruppen im Molekül,

b) ein Organopolysiloxan ohne reaktive Gruppen,

c) ein Organohydrogenpolysiloxan mit zwei oder mehr SiH-Gruppen im Molekül,

d) einen Katalysator zur Beschleunigung der Additionsreaktion, gegebenenfalls

e) Füllstoffe, gegebenenfalls

f) organische Strukturbildner und gegebenenfalls

g) Farbstoffe,

dadurch gekennzeichnet, daß die Massen zusätzlich

h) Compounds von hochdispersen, aktiven Füllstoffen in Silikonöl enthalten.

Komponente c) der oben genannten Rezeptur ist in der Basispaste, Komponente d) in der Katalysatorpaste enthalten. Komponente a) kann entfallen, wenn die Komponente h) ein Vinylgruppen aufweisendes Siloxan enthält. Basis- und Katalysatorpaste der erfindungsgemäßen Vinylsilikon-Abformmassen zeichnen sich durch gute Lagerstabilität hinsichtlich Viskosität und Sedimentation aus; die daraus hergestellten Abformungen weisen gute Reißfestigkeit und Flexibilität bei hohem Rückstellvermögen auf. Basis- und Katalysatorpaste der erfindungsgemäßen Dubliermassen auf Vinylsilikon-Basis weisen eine gute Lagerstabilität und Fließfähigkeit auf: die daraus hergestellten Abformungen besitzen hohe Reißfestigkeit, Weichheit und Flexibilität bei hohen Rückstellvermögen und benötigen keine langen Wartezeiten bis zur Weiterverarbeitung.

Bei dem Silikonöl (a) handelt es sich um an sich bekannte vinylendgestoppte Polydimethylsiloxane, deren Viskosität z.B. im Bereich von 100 bis 100.000 mPa.s bei 20°C liegen kann, je nach gewünschter Konsistenz der formulierten Paste.

Das Silikonöl (b) ist vorzugsweise ein trimethylsiloxyendgestopptes Polydimethylsiloxan mit einem Viskositätsbereich von 50 bis 1.000 mPa.s bei 20°C.

Der Vernetzer (c) ist ein ebenfalls an sich bekanntes Polydimethylsiloxan, welches in seinem Molekül Wasserstoffatome an mindestens zwei Siliciumatomen aufweist.

Bei dem Katalysator (d) handelt es sich z.B. um einen Platin-Komplex, der aus Hexachlorplatin-IV-säure hergestellt wurde. Auch diese Verbindungen sind an sich bekannt.

Unter den Füllstoffen (e) versteht man z.B. Calciumcarbonat, Quarz- und Cristobalitmehle, sowie gefällte und pyrogen hergestellte Siliciumdioxide, deren Oberflächen vorzugsweise mit an sich bekannten, zur Oberflächenmodifizierung dienenden Substanzen, z. B. auf Basis von Silazan, beladen wurden.

Der organische Strukturbildner (f) ist bevorzugt ein hydriertes Rizinusöl, dessen Schmelzbereich vorzugsweise bei 80 bis 95°C, insbesondere bei 82 bis 90°C liegt. Selbstverständlich können jedoch auch andere an sich bekannte Thixotropiermittel verwendet werden.

Bevorzugt werden Farbstoffe (g) zur Unterscheidung der Basis- und Katalysatorpaste und zur Mischkontrolle eingesetzt. Anorganische und organische Farbpigmente werden besonders bevorzugt.

Die erfindungsgemäß einzusetzenden Compounds (h) werden, wie in der DE-OS 25 35 324 beschrieben, aus einer gefällten oder pyrogen hergestellten Kieselsäure mit einer spezifischen Oberfläche von mehr als 50 $m^2/g$ nach BET und einem trimethylsilyl- und/oder vinylendgestoppten Polydimethylsiloxan mit einer Viskosität bevorzugt zwischen 500 und 100.000 mPa.s bei 20°C unter Verwendung eines Modifizierungsmittels, z.B. Hexamethyldisilazan, hergestellt.

Man geht dabei so vor, daß man den Füllstoff während des Einarbeitungsprozesses in Gegenwart von Wasser mit einem Modifizierungsmittel der allgemeinen Formel

$$(R_3Si)_n - X$$

wobei R für einen substituierten oder nicht substituierten gesättigten oder aliphatisch ungesättigten Kohlenwasserstoffrest mit bis zu 10 C-Atomen steht, X Halogen, OH, OR, S, OOCR, N oder NY (Y = Wasserstoff oder R) ist und n 1, 2 oder 3 bedeutet, behandelt.

Die für die erfindungsgemäßen Massen geeigneten Compounds enthalten die folgenden Einzelbestandteile:

1. 20 bis 1000, vorzugsweise 50 - 200 Gewichtsteile eines linearen Polyorganosiloxans der allgemeinen Formel

$$R^1 - \underset{R^2}{\overset{R^2}{Si}} - \left[ O - \underset{R^2}{\overset{R^2}{Si}} \right]_m - O - \underset{R^2}{\overset{R^2}{Si}} - R^1$$

wobei $R^2$ gleiche oder verschiedene einwertige substituierte oder nichtsubstituierte Kohlenwasserstoffreste darstellt, $R^1$ dieselbe Bedeutung wie $R^2$ hat und darüber hinausgehend einen Vinylrest darstellen kann und m eine ganze positive Zahl ist;

2. 5 bis 500, vorzugsweise 10 - 100 Gewichtsteile eines hochdispersen aktiven Füllstoffes mit einer BET-Oberfläche von mindestens 50 $m^2/g$.

Bei dem oben angegebenen Bestandteil 1) handelt es sich um ein lineares Polyorganosiloxan, dessen Molekülkette mit einwertigen Kohlenwasserstoffresten $R^2$ abgesättigt ist. $R^2$ kann ein substituierter oder nichtsubstituierter, gesättigter oder aliphatisch ungesättigter Kohlenwasserstoffrest sein und ist vorzugsweise ausgewählt aus Alkylresten, wie z.B. Methyl, Ethyl, Propyl, Butyl, Hexyl, i-Propyl, Amyl, oder Arylresten, wie z.B. Phenyl, Diphenyl, Naphthyl, oder Alkarylresten, wie z.B. Tolyl, Xylyl, Ethylphenyl, oder Aralkylreste, wie z.B. Benzyl, Phenylethyl oder halogensubstituierten Alkyl- oder Arylresten,

wie z.B. Chlormethyl, 3,3,3-Trifluorpropyl, Chlorphenyl, Tetrachlorphenyl, Difluorphenyl und Alkenylresten, wie z.B. Vinyl, Allyl. Außerdem steht $R^2$ auch für Cyanoalkyl-, Cycloalkyl- und Cycloalkenylreste.

$R^1$ hat dieselbe Bedeutung wie $R^2$, kann darüber hinaus aber auch eine Vinylgruppe darstellen.

In einem Molekül des Bestandteiles 1) können unterschiedliche Reste $R^1$ und $R^2$ vorhanden sein.

Die Größe der Zahl m ist maßgebend für die Viskosität des linearen Polyorganosiloxans und ist so gewählt, daß die Viskosität des linearen Polyorganosiloxans bevorzugt zwischen 10 cP und $8.10^6$ cP bei 25°C liegt. Bestandteil 1) kann auch aus Mischungen von Polymeren mit unterschiedlicher Größe der Zahl m bestehen.

Komponente 2) ist ein handelsüblicher hochdisperser, aktiver Füllstoff mit einer BET-Oberfläche von mindestens 50 $m^2$/g. In Frage kommen Stoffe wie $TiO_2$, $Al_2O_3$, ZnO oder bevorzugt pyrogen gewonnene oder naßgefällte Kieselsäure. Das Herstellungsverfahren und die chemische Natur des Füllstoffes spielen für die erfindungsgemäße Verwendung keine Rolle. Wichtig ist lediglich, daß es sich um einen Füllstoff mit einer BET-Oberfläche in der angegebenen Größenordnung handelt. Vorteilhaft ist ferner, daß kein während oder nach seiner Herstellung modifizierter Füllstoff eingesetzt werden muß, sondern daß handelsübliche Füllstoffe verwendet werden können. Beispielhaft als geeignete Füllstoffe seien genannt pyrogen

gewonnene oder naßgefällte Kieselsäure, die unter den Handelsnamen Aerosil R von Degussa, Cabosil R von Cabot, HDK R von Wacker-Chemie, pyrogen gewonnenes Aliminiumoxid oder Titandioxid unter der Bezeichnung Aluminiumoxid O R bzw. Titandioxid P 25 R von Degussa oder Zinkoxid aktiv R von der Bayer AG angeboten werden.

Die Einarbeitung des hochdispersen aktiven Füllstoffs geschieht gemäß DE-OS 25 35 334 in der Weise, daß der Füllstoff während des Einarbeitungsprozesses in Gegenwart von Bestandteil 1) in geeigneter Form an der Oberfläche modifiziert wird. Dazu ist es notwendig, daß vor der Zugabe des Füllstoffs das Modifizierungsmittel zu Bestandteil 1) zugefügt wird. Bei dem Modifizierungsmittel handelt es sich um eine Substanz, die in der Lage ist, die Oberfläche des Füllstoffs ohne Gegenwart eines zusätzlichen Katalysators in der gewünschten Weise zu modifizieren, die aber nicht mit Bestandteil 1) unter den gewählten Reaktionsbedingungen eine chemische Reaktion eingeht. Es wurde festgestellt, daß Verbindungen der allgemeinen Formel

$$(R_3Si)_nX$$

geeignet sind, Füllstoffe in der gewünschten Weise in Gegenwart von Bestandteil 1) zu modifizieren. Dabei hat R die oben angegebene Bedeutung, n ist eine positive Zahl und kann den Wert 1, 2 oder 3 haben und X ist ein Rest der Formel H, OH, OR, Halogen, S, OOCR, N

oder NY (wobei R die unter a) angegebene Bedeutung hat und Y ein einwertiger Kohlenwasserstoffrest oder Wasserstoff ist). Beispielhaft seien genannt: Hexamethyldisilazan, Trimethylsilan, Trimethylchlorsilan, Trimethylethoxysilan, Triorganosilylacylate oder Triorganosilylamine.

Besonders bevorzugt sind erfindungsgemäß Verbindungen, in denen der Rest R ein Methylrest ist und der Rest X die Gruppe NY darstellt, wobei Y vorzugsweise ein Wasserstoffatom ist.

In der Regel wird das Modifizierungsmittel in einer Menge von 1-50, vorzugsweise 3-20 Gewichtsteilen zugegeben.

Es ist bevorzugt, daß zu der Mischung von Bestandteil 1) und dem Modifizierungsmittel vor Zugabe des hochdispersen aktiven Füllstoffes Wasser in einer Menge von 0,1 bis 10 Gewichtsteilen, vorzugsweise von 2 bis 6 Gewichtsteilen zugefügt wird. Durch die Gegenwart von Wasser wird die Reaktion zwischen Modifizierungsmittel und hochdispersem Füllstoff begünstigt. Zusätzlich trägt das Wasser zu einer beschleunigten gleichmäßigen Verteilung des Füllstoffes in Gegenwart des Modifizierungsmittels bei.

Die Einarbeitung des hochdispersen aktiven Füllstoffes erfolgt bei Raumtemperatur oder nur leicht erhöhter Temperatur. Die Einarbeitung ist unkritisch, und es brauchen keine besonderen Vorsichtsmaßnahmen berücksichtigt zu werden. Es ist zweckmäßig, den hochdispersen aktiven Füllstoff zu der Mischung aus Bestandteil 1), dem

Modifizierungsmittel und Wasser nicht auf einmal, sondern portionsweise zuzugeben, so daß die jeweils zugefügte Menge an hochdispersem aktiven Füllstoff in kurzer Zeit benetzt und eingearbeitet wird. Die Verteilung des hochdispersen aktiven Füllstoffes in der Mischung aus Bestandteil 1), dem Modifizierungsmittel und Wasser kann mit handelsüblichen, dafür geeigneten Geräten erfolgen, vorzugsweise mit sogenannten Z-Knetern oder Planetenrührern.

Die Menge des einzuarbeitenden hochdispersen aktiven Füllstoffes richtet sich nach der gewünschten Konsistenz der Mischung und im Falle von Mischungen, in denen Bestandteil 1) Reste $R^1$ oder $R^2$ enthält, die einer Vernetzungsreaktion bei Raumtemperatur mit entsprechenden Vernetzersubstanzen zugänglich sind, nach den erforderlichen mechanischen Eigenschaften der vernetzten Gummis.

Nach vollständiger Einarbeitung des hochdispersen aktiven Füllstoffes wird das aus Komponente 1), dem Modifizierungsmittel, Wasser und Füllstoff bestehende Gemisch vorzugsweise einer kurz währenden mechanischen Beanspruchung (z.B. Überdruck, Druck zwischen Walzen, Knetbeanspruchung) in der Weise unterworfen, daß das Gemisch 10 Minuten bis 2 Stunden lang im dicht verschlossenen Mischgerät verbleibt. Nach Beendigung der mechanischen Beanspruchung der Mischung wird überschüssiges Modifizierungsmittel und Wasser in der Weise entfernt, daß entweder Vakuum angelegt wird oder das Mischaggregat bei erhöhter Temperatur so lange geöffnet und abgelüftet wird, bis

überschüssiges Modifizierungsmittel und Wasser sich praktisch vollständig verflüchtigt haben. Vorzugsweise werden gleichzeitig die Temperatur erhöht und Vakuum angelegt.

Vorzugsweise enthalten erfindungsgemäße Abformmassen (d.h. das Gemisch aus Basis- und Katalysatorpaste) folgende Mengenanteile (in Gew.-%, bezogen auf gesamte Paste) der einzelnen Komponenten:

a) 0 bis 20 %, insbesondere 0 bis 15 % des Vinylgruppen aufweisenden Polysiloxans;
b) 5 bis 25 %, insbesondere 10 bis 20 %, des gesättigten Silikonöls;
c) 2 bis 25 %, insbesondere 3 bis 15 %, Vernetzer;
d) 0,05 bis 1,0 %, insbesondere 0,01 bis 0,5 %, Katalysator;
e) 10 bis 60 %, insbesondere 25 bis 55 %, Füllstoff;
f) 0,005 bis 2,0 %, insbesondere 0,1 bis 1,5 %, Thixotropiermittel;
g) 0,1 bis 3,0 %, insbesondere 0,5 bis 2,0 %, Farbstoffe und
h) 15 bis 55 %, insbesondere 20 bis 40 %, des erfindungsgemäß zu verwendenden Compounds.

Erfindungsgemäße Dubliermassen (d.h. das Gemisch aus Basis- und Katalysatorpaste) enthalten vorzugsweise folgende Mengenanteile (Gew.-%, bezogen auf gesamte Masse) der einzelnen Komponenten :

a) 0 bis 25 %, insbesondere 1 bis 10 %, des Vinylgruppen aufweisenden Polysiloxans;
b) 20 bis 60 %, insbesondere 30 bis 55 %, des gesättigten Silikonöls;

c) 3 bis 25 %, insbesondere 5 bis 20 %, Vernetzer
d) 0,005 bis 1,0 %, insbesondere 0,01 bis 0,5 %, Katalysator
e) 0,1 bis 5,0 %, insbesondere 0,2 bis 3,0 %, Farbstoffe und
f) 10 bis 65 %, insbesondere 15 bis 55 %, des erfindungsgemäß zu verwendenden Compounds.

Vorzugsweise enthalten die erfindungsgemäß zu verwendenden Compounds 5 bis 50 Gew.-% (bezogen auf gesamtes Compound), insbesondere 15 bis 40 Gew.-%, an hochdispersen, aktiven Füllstoffen.

Insgesamt enthalten die erfindungsgemäßen Abform- und Dubliermassen (aus Komponente a) und Komponente h) bzw. f)) vorzugsweise 10 bis 60 Gew.-%, besonders bevorzugt 20 bis 45 Gew.-%, an Vinylgruppen aufweisenden Polysiloxanen.

Die nachfolgenden Beispiele, in denen alle Teile Gewichtsteile bedeuten, erläutern die Erfindung.

Beispiel 1

In einem Kneter werden 700 Teile vinylendgestopptes Polydimethylsiloxan mit einer Viskosität von 1.000 mPa.s bei 20°C mit 40 Teilen Hexamethyldisilazan und 35 Teilen Wasser gemischt und anschließend mit 225 Teilen einer pyrogen hergestellten Kieselsäure mit einer speziellen Oberfläche von 300 m²/g nach BET zu einer homogenen Masse verknetet. Die Mischung wurde zunächst auf 130°C erwärmt und 1,5 Stunden im geschlossenen Kneter gerührt und danach bei 160°C unter Vakuum 1 Stunde von Wasser und überschüssigem Hexamethyldisilazan befreit. Nach dem Erkalten erhält man einen Vaseline-ähnlichen Compound.

Beispiel 2

Beispiel 1 wurde mit einem vinylendgestoppten Polydimethylsiloxan mit einer Viskosität von 65.000 mPa.s bei 20°C anstatt 1.000 mPa.s bei 20°C wiederholt. Der hergestellte Compound war eine zähe Masse.

Beispiel 3

Die Basispaste einer viskosen Abformmasse wurde hergestellt durch Vermischen in einem Kneter von 170 Teilen des Compounds aus Beispiel 1, 60 Teilen des Compounds aus Beispiel 2, 135 Teilen trimethylsiloxyendgestopptem Polydimethylsiloxan mit einer Viskosität von 120 mPa.s, 120 Teilen dimethylhydrogensiloxy-endgestopptem Polydimethylsiloxan mit einer Viskosität von 120 mPa.s bei 20°C, 500 Teilen Quarzfeinstmehl mit einer mittleren Korngröße von ca. 4 µm, 14 Teilen anorganischem Farbpigment und 1 Teil hydriertem Rizinusöl mit einem Schmelzpunkt von 85°C, wobei die Paste auf 95°C erwärmt und unter Rühren auf 25°C abgekühlt wurde.

Die Herstellung der dazugehörenden Katalysatorpaste erfolgte durch Vermischen von 430 Teilen des Compounds aus Beispiel 1, 50 Teilen des Compounds aus Beispiel 2, 248,8 Teilen trimethylsiloxy-endgestopptem Polydimethylsiloxan mit einer Viskosität von 120 mPa.s bei 20°C, 270 Teilen des oben genannten Quarzfeinstmehls, 1 Teil hydriertem Rizinusöl mit einem Schmelzpunkt von 85°C, wobei die Paste auf 95°C erhitzt und unter Rühren auf 25°C abgekühlt wurde, und 0,2 Teilen einer Komplexverbindung aus Platin und Divinyltetramethyldisiloxan.

Die Pastenviskosität wurde mit dem Rotationsviskosimeter der Fa. Haake, bestehend aus Platte-Kegel-Einrichtung PK 100 Meßkopf M 500 und Schreiber RV 100 mit entsprechenden Prüfprotokollen (Bestellnummer 222-0068) gemessen, wobei die Basispaste eine Viskosität von 39.200 mPa.s bei 23°C und die Katalysatorpaste 40.700 mPa.s bei 23°C aufwies. Nach einer Lagerung bei 23°C über 2 Monate wurden Viskositäten von 40.300 mPa.s bzw. 42.100 mPa.s gemessen. Beide Pasten zeigten nach einem Zentrifigugentest (1 Stunde, 12.000 UpM) keine Sedimentation.

5 g Basis- und 5 g Katalysatorpaste wurden auf einem Anmischblock mittels Spatel innerhalb von 30 Sekunden gut gemischt. Die Prüfung der elastischen Verformung und bleibenden Deformation erfolgte nach der Spezifikation Nr. 19 der American Dental Association und ergab Werte von 6,7 % und 0,17 %. Die Härte betrug 40° Shore A (10 Min. nach Mischbeginn), die Reißfestigkeit wurde gut beurteilt.

Beispiel 4 (Vergleichsbeispiel)

In einem Kneter wurde die Basispaste hergestellt durch Vermischen von 340 Teilen vinylendgestopptem Polydimethylsiloxan mit einer Viskosität von 10.000 mPa.s bei 20°C, 210 Teilen dimethylhydrogensiloxy-endgestopptem Polydimethylsiloxan mit einer Viskosität von 120 mPa.s bei 20°C, 370 Teilen des Quarzfeinstmehles aus Beispiel 3, 60 Teilen gefällter und oberflächlich silanisierter Kieselsäure mit einer spezifischen Oberfläche von 90 $m^2/g$ nach BET und 20 Teilen anorganischem Farbpigment.

Die Katalysatorpaste wurde in einem Kneter hergestellt durch Vermischen von 548 Teilen vinylendgestopptem Polydimethylsiloxan mit einer Viskosität von 5000 mPa.s bei 20°C, 390 Teilen Quarzfeinstmehl aus Beispiel 1, 60 Teilen der obengenannten Kieselsäure, 1,8 Teilen Titandioxid und 0,2 Teilen des Platin-Siloxankomplexes von Beispiel 3.

Die Viskosität der Basispaste betrug nach der Herstellung 42.700 mPa.s bei 23°C, die der Katalysatorpaste 44.600 mPa.s bei 23°C. Die Viskositätswerte stiegen nach einer Lagerung von 2 Monaten bei 23°C auf 53.200 mPa.s bzw. 56.900 mPa.s an. Nach dem Zentrifugentest konnten bei beiden Pasten Füllstoff-Sedimentationen beobachtet werden.

Die Prüfung des aus der Basis- und Katalysatorpaste hergestellten Gummis ergab folgende Werte: 3,2 % elastische Verformung, 0,29 % bleibende Deformation und 45° Shore A; die Reißfestigkeit wurde als befriedigend beurteilt.

Beispiel 5

Die Basispaste einer Dubliermasse wurde hergestellt durch Vermischen in einem Kneter von 200 Teilen des Compounds aus Beispiel 1, 350 Teilen des Compounds aus Beispiel 2, 350 Teilen trimethylsiloxy-endgestopptem Polydimethylsiloxan mit einer Viskosität von 100 mPa.s bei 20°C und 100 Teilen dimethylhydrogensiloxy-endgestopptem Polydimethylsiloxan mit einer Viskosität von 120 mPa.s bei 20°C.

Die Herstellung der dazugehörenden Katalysatorpaste erfolgte in einem Kneter durch Vermischen von 200 Teilen des Compounds aus Beispiel 1, 350 Teilen des Compounds aus Beispiel 2, 430 Teilen trimethylsiloxy-endgestopptem Polydimethylsiloxan mit einer

Viskosität von 100 mPa.s bei 20°C, 19 Teilen anorganischem Farbpigment und 1 Teil Platin-Siloxan-Komplex aus Beispiel 3.

Die Viskositätsmessung ergab für die Basispaste 11.600 mPa.s bei 23°C und für die Katalysatorpaste 7.800 mPa.s bei 23°C unmittelbar nach der Herstellung und 12.600 mPa.s bzw. 8.600 mPa.s bei 23°C nach einer Lagerung über 2 Monate bei 23°C. Nach den Zentrifugentest konnte keine Sedimentation festgestellt werden.

9 g Basispaste und 1 g Katalysatorpaste wurden auf einem Anmischblock mittels Spatel gemischt. Der entstandene Gummi wies 15 Minuten nach Mischbeginn eine Härte von 12° Shore A auf, war somit weich und flexibel und besaß eine hohe Reißfestigkeit und ein hohes Rückstellvermögen.

Ein zu vervielfältigendes Gipsmodell wurde in eine Dublierküvette gestellt und mit einer unter Vakuum hergestellten Mischung von 270 g Basispaste und 30 g Katalysatorpaste eingegossen. 15 Minuten später konnte das Gipsmodell problemlos ausgeformt werden, da die Dubliermasse weich und flexibel war und nicht einriß. Danach konnte sofort eine wäßrige Gipsaufschlämmung in die Abformung gegossen werden und nach der Aushärtung des Gipses glich diese Kopie dem "Urmodell" auf das genaueste.

Patentansprüche

1. Additionsvernetzende Massen auf Polysiloxanbasis, enthaltend

   a) ein Organopolysiloxan mit zwei oder mehreren Vinylgruppen im Molekül,

   b) ein Organopolysiloxan ohne reaktive Gruppen,

   c) ein Organohydrogenpolysiloxan als Vernetzer,

   d) einen Katalysator zur Beschleunigung der Additionsreaktion

   e) gegebenenfalls anorganische Füllstoffe, gegebenenfalls

   f) organische Strukturbildner und gegebenenfalls

   g) Farbstoffe,

   dadurch gekennzeichnet, daß sie zusätzlich Compounds von hochdispersen Füllstoffen in Silikonölen enthalten, wobei Komponente a) ganz oder teilweise im Compound enthalten sein kann.

2. Massen nach Anspruch 1, dadurch gekennzeichnet, daß der Compound aus einem trimethylsilyl- und/oder vinylendgestoppten Polydimethylsiloxan mit einer Viskosität zwischen 100 und 100.000 mPa.s

bei 20°C und einer gefällten oder pyrogen hergestellten Kieselsäure mit einer spez. Oberfläche von mindestens 50 m²/g nach BET besteht.

3. Massen nach Anspruch 1 oder 2, dadurch gekennzeichnet, daß der Compound in Gegenwart von Wasser und Hexamethyldisilazan als Modifizierungsmittel hergestellt wurde.

4. Massen nach Anspruch 1 bis 3, dadurch gekennzeichnet, daß der Compound 5 bis 50 Gew.-%, insbesondere 15 bis 40 Gew.-% (bezogen auf gesamten Compound) an hochdispersem, aktivem Füllstoff enthält.

5. Dental-Abformmasse nach Anspruch 1 bis 4, dadurch gekennzeichnet, daß sie folgende Mengenanteile der einzelnen Komponenten, bezogen auf gesamte Paste, enthält:

| | | | | | |
|---|---|---|---|---|---|
| a) | 0 | bis | 20 | Gew.-% | des Vinylgruppen aufweisenden Polysiloxans; |
| b) | 5 | bis | 25 | Gew.-% | des Organopolysiloxans ohne reaktive Gruppen; |
| c) | 2 | bis | 25 | Gew.-% | Vernetzer; |
| d) | 0,005 | bis | 1,0 | Gew.-% | Katalysator; |
| e) | 10 | bis | 60 | Gew.-% | Füllstoff; |

| | | | | | |
|---|---|---|---|---|---|
| f) | 0,05 | bis | 2,0 | Gew.-% | organische Strukturbildner; |
| g) | 0,1 | bis | 3,0 | Gew.-% | Farbstoffe und |
| h) | 15 | bis | 55 | Gew.-% | des Compounds. |

6. Masse nach Anspruch 5, dadurch gekennzeichnet, daß sie folgende Mengenanteile der einzelnen Komponenten, bezogen auf gesamte Paste, enthält:

| | | | | | |
|---|---|---|---|---|---|
| a) | 0 | bis | 15 | Gew.-% | des Vinylgruppen aufweisenden Polysiloxans; |
| b) | 10 | bis | 20 | Gew.-% | des Organopolysiloxans ohne reaktive Gruppen; |
| c) | 3 | bis | 15 | Gew.-% | Vernetzer; |
| d) | 0,01 | bis | 0,5 | Gew.-% | Katalysator |
| e) | 25 | bis | 55 | Gew.-% | Füllstoff; |
| f) | 0,1 | bis | 1,5 | Gew.-% | organische Strukturbildner; |
| g) | 0,5 | bis | 2,0 | Gew.-% | Farbstoffe und |
| h) | 20 | bis | 40 | Gew.-% | des Compounds. |

7. Dental-Dubliermasse nach Anspruch 1 bis 4, dadurch gekennzeichnet, daß sie folgende Mengenanteile der einzelnen Komponenten, bezogen auf gesamte Paste, enthält:

| | | | | | |
|---|---|---|---|---|---|
| a) | 0 | bis | 25 | Gew.-% | des Vinylgruppen aufweisenden Polysiloxans; |
| b) | 20 | bis | 60 | Gew.-% | des Organopolysiloxans ohne reaktive Gruppen; |
| c) | 3 | bis | 25 | Gew.-% | Vernetzer; |
| d) | 0,005 | bis | 1,0 | Gew.-% | Katalysator; |
| e) | 0,1 | bis | 5,0 | Gew.-% | Farbstoffe und |
| f) | 10 | bis | 65 | Gew.-% | des Compounds. |

8. Masse nach Anspruch 7, dadurch gekennzeichnet, daß sie folgende Mengenanteile der einzelnen Komponenten, bezogen auf gesamte Paste, enthält:

| | | | | | |
|---|---|---|---|---|---|
| a) | 1 | bis | 10 | Gew.-% | des Vinylgruppen aufweisenden Polysiloxans; |
| b) | 30 | bis | 55 | Gew.-% | des Organopolysiloxans ohne reaktive Gruppen; |
| c) | 5 | bis | 20 | Gew.-% | Vernetzer; |

d) 0,01 bis 0,5 Gew.-% Katalysator;

e) 0,2 bis 3,0 Gew.-% Farbstoffe und

f) 15 bis 55 Gew.-% des Compounds.

9. Verwendung von 5 bis 50 Gew.-%, vorzugsweise 15 bis 40 Gew.-%, an hochdispersen aktiven Füllstoffen in Silikonölen enthaltenden Compounds als Füllstoffkomponente in additionsvernetzenden Silikonmassen.

10. Verwendung nach Anspruch 9 in Abform- oder Dubliermassen für Dentalzwecke.

**0169365**

Nummer der Anmeldung

EP 85 10 7424

Europäisches Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | US-A-3 950 300 (P. HITTMAIR et al.)<br>* Ansprüche 1,2,10-16; Spalte 4, Zeilen 6-11,39-59; Spalte 5, Zeilen 64-68; Spalte 6, Zeilen 1-24 *<br>--- | 1,2 | C 08 L 83/07 //<br>A 61 K 6/10<br>(C 08 L 83/07<br>C 08 L 83:05 ) |
| A | EP-A-0 046 907 (BAYER AG)<br>* Anspruch 1; Seite 6, Zeilen 1-3; Seite 10, Beispiel 1 *<br>--- | 1 | |
| A | DE-A-2 313 218 (WACKER-CHEMIE GmbH)<br>* Ansprüche 1-2 *<br>--- | 1 | |
| A | FR-A-2 277 848 (WACKER-CHEMIE GmbH)<br>* Anspruch 1 *<br>----- | 1 | RECHERCHIERTE SACHGEBIETE (Int. Cl.4)<br>C 08 L<br>A 61 K |

Der vorliegende Recherchenbericht wurde für alle Patentansprüche erstellt

| Recherchenort | Abschlußdatum der Recherche | Prüfer |
|---|---|---|
| DEN HAAG | 05-09-1985 | DEPIJPER R.D.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82